# EUROPEAN PATENT APPLICATION

(11) **EP 4 841 296 A1**
(43) Date of publication of application: **18.11.2026**
(21) Application number: 25176036.9
(22) Date of filing: 13.05.2025
(51) Int. Cl.: G01S 7/41, G01S 13/536, A61B 5/02, A61B 5/05, A61B 5/08, G01S 13/00

(54) **HOLOGRAPHIC SONIC MICROWAVE CAMERA FOR MOVING OBJECTS**

(71) Applicant: Lepple-Wienhues, Albrecht, 25300 Pontarlier (FR)
(72) Inventor: Lepple-Wienhues, Albrecht, 25300 Pontarlier (FR)

(57) **Abstract**

A device for providing a non-contact, time-resolved, three-dimensional, continuous scan of at least one structural element of an object which uses microwaves, comprising of at least one antenna element, at least one transmitter and at least one receiver, at least one comparator for comparing emitted microwaves and microwaves which are reflected from said element, and at least one analyzer for analyzing at least one property of the emitted microwaves and the received microwaves selected from the group of frequency, phase, polarization and amplitude.

Further the device comprises at least one control and/or processing unit designed for sampling two-dimensional images at a high repetition rate in which each image pixel contains information about the relative distance and/or velocity of said structural elements towards the antenna elements. The invention thus allows a repeated spatial reconstruction of structural elements in an object with sufficient scan rate to characterize the frequency of the vibration or speed of the object at one or multiple structures of the object.

## Description

### Field of the invention

The present invention relates to a device for providing a non-contact, three-dimensional scan at a high sampling rate of at least one structural element of moving or vibrating objects. Further, the invention relates to a method for providing data from at least one structural element of a moving or vibrating object using microwaves.

### Background

Radar stands for "RAdio Detection And Ranging". Ranging uses Time Of Flight (TOF) measurement in order to determine distance (range) between the transceiver and the object of interest. To this end, time information must be encoded in the microwave signal. Accurate time information requires a large bandwidth (BW) for high precision of the TOF / range measurement. TOF measurement inherently limits the time resolution, leading to limited scan rates, dwell time and blind velocities. Furthermore, unwanted reflections from static objects including ground, building structures, etc., can obscure the return of interest and limit sensitivity.

Even so-called non-scanning Radar devices rely on time-resolved frequency modulation in order to determine TOF and range when reconstructing a spatially resolved image.

Imaging radar relies on TOF as well as intensity and phase information in order to reconstruct a multi-dimensional image of objects by triangulation. As an example, SAR radar can use phase information on top of TOF for higher range and AOA resolution. However, the scan rate is limited by the repetitive nature of the required signal modulation and the scanning process.

There are, however, applications, where distance / range information is of only secondary concern. Examples for those applications include, but are not limited to medical radar analyzing body surface vibrations, collision detection, bird strike prevention, drone detection and directional localization, surface vibration / flutter technical analysis and surveillance on motors, wind turbines, airplanes, building structures, seismic movement in landslides, glaciers and volcanoes.

All these and many other applications have in common the need for immunity to static reflectors. Clutter immunity and / or removal has priority over range determination. Only moving or vibrating targets are of interest, while static targets should be filtered out.

For example, using a medical radar scanning spatially distributed body surface vibrations, the range can be pre-determined by an appropriate setting for the examination, and only the living object of interest be placed in the field of view. Physiological motions like arterial pulsations allow for precise detection and analysis of anatomical body regions.

For another example, bird strike prevention in airplanes by radar is hindered by very small radar cross section of targets. Thus, sensitivity must be very high. However, high sensitivity in turn increases clutter from unwanted targets including ground and precipitation, thus obstructing target detection and discrimination. Properties of main interest to discriminate birds from other targets are wing flapping, relative speed against the airplane, and angle of arrival (AOA) for localization. Similar preferences apply to drone detection and localization, where typical vibration frequencies from rotors and engines can be used to discriminate drones from other targets.

Continuous wave (CW) radar with a single beam has long been used for vehicle speed measurement. While range / distance determination in this application is not required, the operator must ensure a sufficiently narrow beam being precisely aligned in order to avoid Doppler recording of unwanted targets.

A similar radar has been used in the medical field, yielding touchless physiological signals like pulse and breathing movements through a CW single radar beam reflected by the skin and using interferometry to extract the physiological movements and vibrations typically in the acoustic and sub acoustic range.

While a CW device provides no absolute distance / range information directly, it can detect phase modulation by movement of the reflecting target, resulting in phase shifts at the receiver over time. The vibrating or moving target is permanently changing the relative distance/range. A vibrating motion target is thus phase-shifting the reflected microwave in an oscillatory pattern. Continuous motion targets are phase-shifting the received microwave via the Doppler effect. In any case, the received microwave contains the reflectors mechanical frequency or doppler frequency, allowing for filtering out static or other unwanted targets and to focus on typical vibration frequency and velocity bands combined with AOA determination.

Although medical radar has advantages over classical auscultation with a stethoscope (e.g. penetration through clothing and bedding), its use has so far been mostly limited to research. The reason is the microwave beam illuminating large body surfaces like the chest wall, therefore lack of precise anatomical location / pathophysiological relevance, and the need for trained medical personnel directing the beam correctly and interpreting the resulting data according to the targeting of the beam. Therefore, a three-dimensional radar scan would greatly improve usability and clinical relevance, eliminating the need for a specialized examiner present during the examination.

Imaging radar trying to penetrate the body for e.g. tumor detection using antennas galvanically connected to the skin is a fundamentally different concept and therefore omitted from this discussion of medical radar.

Recently, spatially resolving medical RADAR has been proposed (EP3781015). However, using TOF - based radar the dependency of triangulated lateral resolution on microwave BW requires the use of a large BW at high microwave frequencies, complicating the design at increased cost, while at the same time sacrificing microwave penetration capabilities through bedding and clothing and adding sensitivity to body surface beam incidence angle. Furthermore, the time resolution / scan rate is inherently limited.

The present invention overcomes these and other limitations, by using for the first time interferometric CW radar in a spatially resolved, holographic manner. The invention allows for precise spatial resolution, high frame rate at minimal microwave BW, and cost-effective designs at low microwave frequencies using software defined radio. This invention takes full advantage of minimal atmospheric attenuation and good penetration of e.g. bedding and clothing at low microwave frequency bands, eliminates the need for trained medical specialist present during examination, and can benefit from diffuse microwave reflectivity due to wavelengths in the centimeter range. The present invention thus provides a medical scanner for physical examination without the presence of trained medical personnel, useful in telemedicine and health monitoring. Similar advantages apply to other applications outside the medical field.

EP3781015 describes the medical need for and advantages of a spatially resolved medical microwave scanner:
"Many pathologic conditions of the human body reveal specific sounds or pulsations at specific body locations. These include, but are not limited to: arterial pulse wave velocity related to blood pressure, Arrhythmias, Heart valve stenosis/regurgitations, Aortic stenosis, Arterial stenosis (carotid, etc), Aortic aneurysm, Cardiac insufficiency, Pneumonia, Asthma, Pneumothorax, Sleep apnea, Pleuritis, Inborn heart defects, Gastrointestinal atresias, Ileus (paralytic/stenotic), Hyperperistaltic (enteritis), Fetal heart beat, uterine contractions, Restless legs, Tremor, Paralysis"....
M. T. Ghasr et al., "Wideband Microwave Camera for Real-Time 3-D Imaging", IEEE TRANSACTIONS ON ANTENNAS AND PROPAGATION, IEEE SERVICE CENTER, PISCATAWAY, NJ, US, vol. 65, no. 1, January 2017, pages 258-268, describes a microwave video rate camera for time-resolved imaging in security scanners. The 3D information about the target is obtained using time-of-flight triangulation according to the SAR principle.

The disadvantages of those TOF-based approaches include the required high microwave BW for acceptable resolution, since the minimum distance S that can be resolved is inversely proportional to the BW: $S = c / \left(2BW\right)$

In order to achieve e.g. a medically relevant triangulated lateral resolution in the centimeter range, large microwave BW and the use of high frequencies above 10 GHz, e.g. 24 GHz or even 67 GHz are required in order to stay within regulatory and technical band limits. Another disadvantage of high microwave frequency bands lies in reduced penetration through clothing and bedding and atmospheric attenuation. Furthermore, the repetitive scanning proposed in both publications limits the frame rate, i.e. the repetition rate of scanned images. Most medically relevant body surface vibrations lie in the sub acoustic to acoustic range up to 1 kHz, e.g. vascular and respiratory noises. Therefore, according to the Nyquist sample theorem a frame rate of > 2 kHz is desirable for this application example. This high scan repetition rate is not technically achievable using the approaches in Ghasr et al. or EP3781015. Both proposals are restricted to still objects and suffer from limited time resolution.

Furthermore, the required use of high microwave frequencies makes the electronics more costly because special materials like ceramics must be used and special HF circuitry must be highly integrated. Furthermore, atmospheric absorbance is a severely limiting factor at high frequencies and requires the use of large transmission power that may not be desirable or acceptable, e.g. for safety and regulatory reasons. Specular reflection by the target surface renders the reflected beam intensity vulnerable to incidence angle at the target, restricting practical usability.

### Invention and Solution

In view of the shortcomings of the prior art, it is the object of the present invention to develop a new device comprising a CW radar with two-dimensional Phase Difference Of Arrival (PDOA) analysis allowing a sonic microwave holographic scan, i.e. a continuous, non-contact, three-dimensional scan, enabling spatially resolved movement analysis. Since the information is contained in the phase of the microwave modulated by the target, there is virtually no limitation to the scan rate / time resolution.

In a medical application, this represents a scan of at least one anatomical element of a living body, providing physiological and pathological diagnostic information. Similar principles apply with other applications and the examples given here for the medical application do not limit the scope of the invention. Examples for similar applications include, but are not limited to collision detection, detection and localization of birds and flying objects, surface vibration / flutter technical analysis and surveillance on motors, wind turbines, airplanes, building structures, or seismic activity in landslides, glaciers, and volcanoes.

At least this object is solved by the device with the features of independent claim 1 and by the method with the features of independent claim 10. Preferred embodiments of this device and this method are defined in the dependent claims. The wording of all claims is hereby incorporated into this description by explicit reference.

In the present application we disclose a device using radar 3D interferometry with PDOA. The device provides three-dimensional scans at a high sample rate with sufficient lateral resolution and very high resolution for relative distance / range changes over time, allowing the recording of acoustic and sub-acoustic vibrations and movements in real time and at a high image resolution for the entire surface of objects illuminated by the microwave beam.

Surprisingly, when using the present invention, it is not required to perform TOF measurements avoiding the disadvantages described above. Rather, a CW radar uses very limited BW. The vibration and movement of illuminated body surface areas of a subject or reflective object is analyzed interferometrically by comparing the phase of the reflected waves with the transmitted waves. Instead of measuring absolute distance for spatial triangulation, the present invention reveals relative distance changes over time. The spatial resolution is achieved by PDOA analysis of the transmitted and received waves, using geometric phase shift information. The invention thus focusses on the relevant information in many environments including medical examination, i.e. the relative distance changes over time rather than absolute distance measurement, dropping reflections stemming from static reflectors.

By omitting TOF measurements, the invention reduces the BW requirements by an order of magnitude over the state of the art. This allows for a very high frame rate / time resolution since the spatial scan is continuous instead of sequential / repetitive.

While CW does not yield absolute range information at first hand, distance measurements can be obtained secondarily if required. Once the location of an object of interest is known via AOA, distance can be obtained among other means through parallax triangulation via a second linked separate detector, at close range by optimizing autofocus correction of planar wavefront arithmetic, by trigonometrically analyzing speed and AOA track over time, by a narrow segment scan using a TOF resolving radar mode, or alternative distance measurement means, e.g. laser-based distance measurement.

CW radar permits using low local oscillator frequencies in the acoustic range and employment of software-defined radio (SDR) as well as simpler and cheaper components, that are readily available from Wifi and Bluetooth mass produced consumer devices. Cost and complexity of the hardware is thus reduced. The construction of antenna elements is simplified as well.

After downmixing, SDR can be used to perform the entire filtering, frequency shifting and data processing. SDR allows convenient and repeatable calibration of the radar device, Especially phase calibration can be fully automated and performed completely in software.

Other advantages of the present invention arise from the simplicity of the hardware and the enabling and flexibility of SDR. The devices can be made very small and light weight, and at low cost, facilitating widespread use and distribution.

The use of lower microwave frequencies with reduced BW provides better penetration through clothing and bedding and reduced atmospheric absorption. Power requirement is reduced, increasing patient safety and reducing exposure to electromagnetic radiation as well as crosstalk at high TX power.

Diffuse reflectivity is prevalent reducing greatly the influence of incidence angle. Since no specular reflectivity is involved at cm wavelengths and the typical surface structure of nonplanar reflectors, a wide range of incidence angles can be tolerated.

### Detailed description of the invention

CW radar with PDOA analysis can be achieved using several different technical means, e.g. multiple in multiple out (MIMO) transmitter (TX) and receiver (RX) elements, multiple RX elements only, or moving TX and RX elements as in SAR radar. In this description we describe, as an example, a MIMO approach. However, similar principles apply with other variants of radar configuration and this example does not limit the scope of the invention.

The device and method according to the invention use, in a preferred embodiment, electromagnetic waves at frequencies ranging from 0.5 GHz to 100 GHz. For an array of TX antennas distinct microwave signals are generated for each antenna by mixing a local oscillator (LO) microwave signal with different acoustic intermediate frequencies (IF, e.g. 4 - 100 kHz) at least two times the desired acoustic sampling frequency (e.g. 0 - 2 kHz). Thus, different TX frequencies are created in a very narrow band (e.g. 5.8 GHz +/- 4 - 100 kHz). On the receiver side, an array of RX antennas is connected to mixer elements and the signal is mixed with the phase-coherent LO signal. All possible TX/RX pairs comprise virtual RX/TX elements that are spaced for later geometric analysis of phase. The resulting intermediate frequencies are in the high acoustic range and can be digitized with an AD converter at an appropriate sample rate (e.g. 200-2000 kHz). All subsequent processing including filtering and phase calibration can then be performed in the digital domain by software.

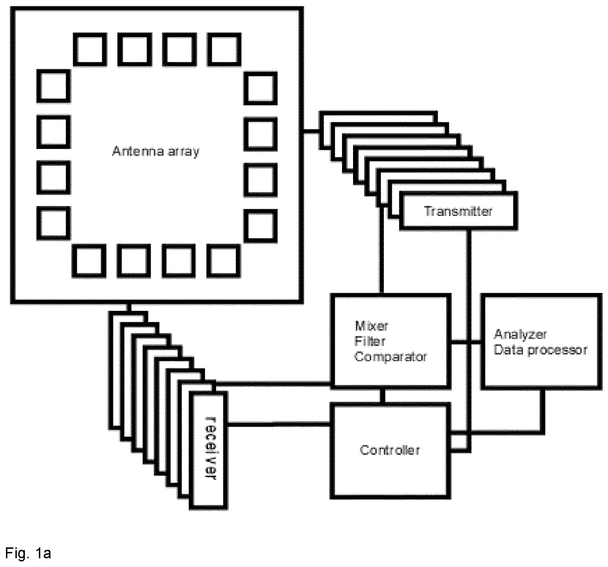

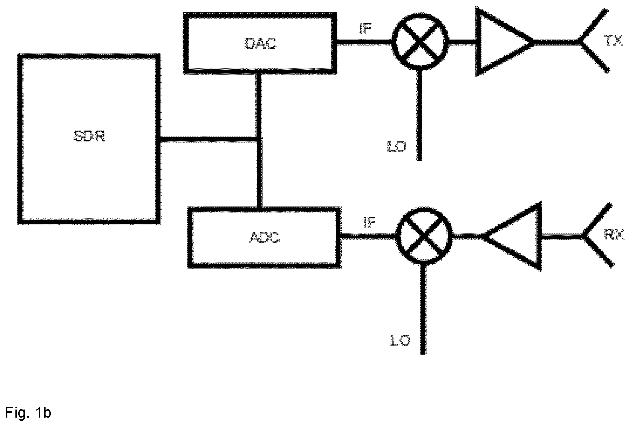

The signals of individual virtual TX/RX elements can then be obtained by frequency shifting and filtering of the down-mixed signals received by the physical RX antenna array. Fig. 1 shows a block diagram of the elements of the CWPDOA radar. This results in time-resolved recordings containing individual phase information for all virtual RX/TX pairs.

A variety of mathematical means exist in order to extract the AOA information as well as the interferometric and / or doppler measurement of reflector displacement over time. Examples include 3D fourier transform and back projection and / or MUSIC algorithms, among others.

Interferometric measurement of reflector displacement over time is achieved by comparing the phase difference of the emitted and the reflected continuous microwave beam. AOA information and image reconstruction is obtained by analyzing the systematic phase displacement between virtual TX/RX elements along azimuth and elevation. Thus, from AOA an image can be reconstructed that contains reflector displacement information over time for each image pixel. No frequency-, code- or amplitude modulation nor TOF measurements are required. No switching between antenna elements limits the continuous flow of phase information. The only limitation for time resolution lies in the chosen IF and corresponding sample rate of the AD converter. Therefore, an interferometric video can be constructed, containing micrometric displacement information for each image pixel at a very high time resolution / frame rate (e.g. 1-20 kHz). This represents an array of virtual stethoscopes / microphones / doppler sensors at high spatial resolution suited to record sub-acoustic and acoustic displacements (e.g. 0 - 2 kHz) for each image pixel. The resolution for relative target movement is mainly limited by the wavelength and the phase noise of the oscillators and lies preferably in the micrometric to submicrometric range (100 nm - 100 um). The cross-range resolution is mainly determined by the number and geometry of antenna elements in the array.

In a preferred embodiment, the device measures arterial pulse wave velocity. In radar scans of the body the outline of the body as well as the heart region and the inguinal regions are detected and the time delay in the arterial pulse between different anatomical regions is determined along with the distance between to two measuring points, enabling the calculation of the arterial pulse wave travel time and corresponding velocity.

The device according to the invention can provide various diagnostic data representing a physical examination detecting a variety of parameters or symptoms. In the preferred embodiments below some examples are given. These examples are not exhaustive and not limiting. It will be clear to one skilled in the art that access to these data will be useful in detecting a wide range of disease states and these examples are not limiting to the diagnostic capabilities of the device of the invention. It is likewise clear to one skilled in the art that a device capable of detecting vibrations in the range given, in an imaging format, can detect numerous pathological states in a manner similar to physical examination including auscultation.

The analysis includes anatomic region, frequency spectrum and sound patterns. For example, there are typical breathing noises over certain lung areas associated with pneumonia or asthma.

In a preferred embodiment, anatomical regions of living objects are recognized, located and mapped. In a preferred embodiment, spatial recognition is based on the inherent distribution of frequencies detected by the device which is, in turn, recognized by software image analysis or artificial intelligence routines. In another preferred embodiment, there is a secondary imaging or mapping modality that supports the mapping of frequency returns.

In a preferred embodiment sounds of stenosis of arteries can be detected. In another preferred embodiment, the device records parameters continuously under exercise or load such that progressive effects on blood pressure can be detected.

In a preferred embodiment asymmetric muscle activity following a stroke and tremors as well as other neurological symptoms are detected.

In a preferred embodiment the device is used for non-contact tocographic monitoring of pregnant women recording uterine contractions and fetal heartbeat from abdominal vibrations.

In a preferred embodiment the device is used outside a medical examination and ambulatory telemedicine, e.g. for home monitoring. The device can perform sleep monitoring, including respiratory and cardiovascular parameters, muscle activity and eye movement

In a preferred embodiment the scanned images are analyzed by machine learning, automated pattern recognition and other elements of artificial intelligence.

The device can also be used in a preferred embodiment in environments outside of the medical field, where spatially resolved relative movement of objects, or the distribution or propagation of vibrations is required or useful.

In a preferred embodiment, surface vibrations of machinery including e.g. engines, airplane wings etc. are analyzed.

In another preferred embodiment, the inner compartment of vehicles is analyzed e.g. for presence of children, alertness, fatigue, or health status of the driver or pilot or other occupants.

In another preferred embodiment autonomous or semi-autonomous vehicles can be equipped with the device in order to analyze the environment and detect e.g. collision hazards with other objects including vehicles or subjects. In a preferred embodiment pedestrians, bicyclists and animals can be located and further characterized using physiological signature signals including pulse and breathing. In these preferred embodiments the narrow BW required by the invention is especially useful, because multiple vehicles equipped with radar sensors are competing for available BW. In a preferred embodiment, the device detects BW in use by neighboring sources and frequency hops to a BW range with optimal signal to noise relationships.

In a preferred embodiment birds are detected and localized in order to prevent bird strikes on airplanes.

In a preferred embodiment flying objects, rotorcraft or other vibrating or rotating airborne objects are detected and localized.

In another preferred embodiment, at least one TX or RX antenna is moved to synthesize an enlarged aperture, multiplying the information received in order to triangulate the positions of reflective elements.

In another preferred embodiment, at least one TX or RX antenna is multiplied in order to triangulate the positions of reflective elements.

In another preferred embodiment, the device is constructed as passive radar, where background microwave illumination is used for analyzing phase information. The microwave illumination can stem from data transfer TXs, e.g. Wifi or Bluetooth, or TXs for radio-controlled devices or other radar sources. The illuminating signal phase is received and phase-referenced by the device, replacing and / or supplementing the phase-coherent TXs in the device.

Further advantages and features of the invention will become clear from the following description of the drawings. The individual features can be incorporated either singly or jointly in combination in one embodiment of the invention. The drawings only serve for illustration and better understanding of the invention and are not to be understood as in anyway limiting the invention.

The drawings schematically show:
Fig. 1a shows a schematic block diagram of the device according to the invention, comprising an antenna array, sending and receiving microwaves. The reflected waves from the range are analyzed in a vertical and horizontal resolution. Phase shift over time is obtained in order to achieve a high motion record when targets move or vibrate. For each image pixel a range movement information over time is extracted allowing for vibration analysis on different image regions. Each TX / RX antenna is connected to its respective TX / RX circuitry.
Fig. 1b gives an example for simplified TX and RX elements, with local oscillator (LO) mixers, DAC and ADC converters for intermediate frequency (IF), and low noise amplifiers. In the RX path DAC converters directly digitize the intermediate frequency (IF), while IF is created by ADC converters for TX. Additional mixers, filters, comparators, analyzers and control elements are all realized digitally in software defined radio (SDR).

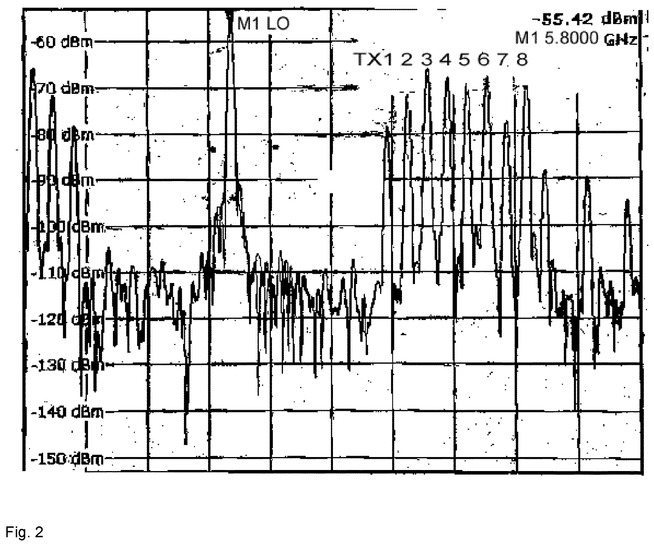

Fig. 2 shows the frequency spectrum of a CW beamforming radar, demonstrating the narrow BW required. The example is using 8 TX channels. The LO at 5.8 GHz is marked with M1. The 8 different TX frequencies are spaced at 3 kHz. Grid width is 10 kHz.

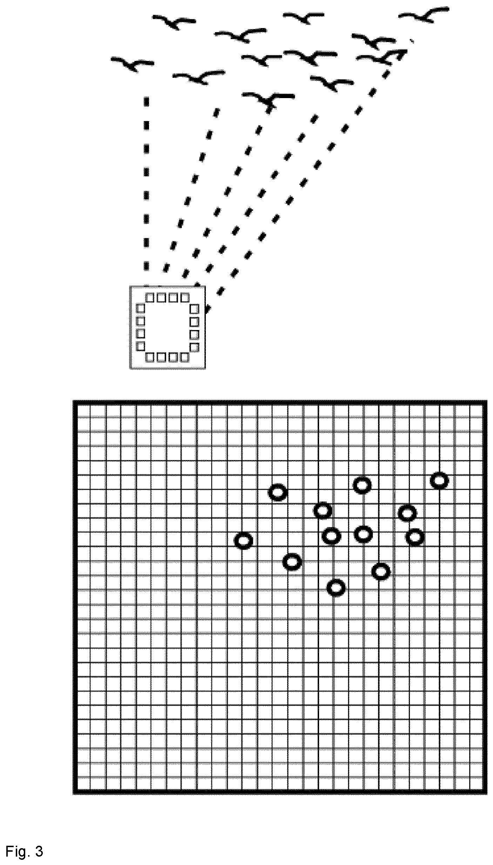

Fig. 3 shows an embodiment of the invention comprising detection and localization of birds. While the phase signal is analyzed for azimuth and elevation, the typical motion frequency band of the reflective target, e.g. wing-flapping, is selected by filters. Thereby, clutter from other reflectors is excluded and the targets of interest can be exclusively localized and tracked.

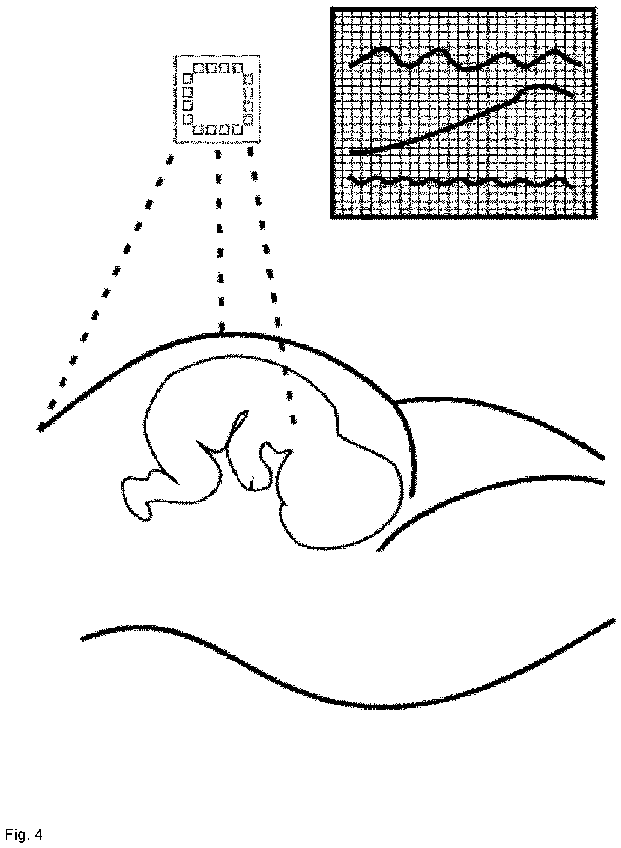

Fig. 4 shows cardiotocographic no-contact recordings in a pregnant woman. Filtered records of maternal heartbeat, uterine contractions and fetal heartbeat are originating with maxima from different anatomical regions.

## Claims

1. Device for providing a non-contact, time-resolved three-dimensional scan of at least one reflective element of an object, using microwaves, preferably microwaves with frequencies between 0.5 GHz to 100 GHz, comprising
- at least one antenna or antenna element,
- at least one transmitter for emitting microwaves,
- at least one receiver for receiving microwaves,
- at least one comparator for comparing emitted microwaves and received microwaves, reflected from the said elements,
- at least one analyzer for analyzing at least one property of the emitted microwaves and the received microwaves selected from the group of parameters comprising frequency, phase, polarization and amplitude, and
- at least one control and/or processing unit designed for continuously sampling from the said compared and analyzed microwave derived two-dimensional images in which each pixel contains information about the precise distance change ΔR over time and/or velocity v of said structural element towards said at least one antenna or antenna element, wherein for each pixel of said images the functions over the time of distance change ΔR(t) and/or velocity v(t) can be calculated, resulting in a repeated spatial reconstruction of said structural element of said object.

2. Device according to claim 1, wherein said analyzer and/or processing unit is designed for analyzing the phase of a portion of a microwave signal representative of at least one pixel of the surface of a reflecting object, wherein said phase analysis allows calculation of surface movement and speed of said at least one pixel with a resolution of 50 um or less, or with a resolution of 10 um or less, or with a resolution of 1 um or less.

3. Device according to claim 1 or 2, wherein the device is configured for analyzing microwave reflections from a surface and/or from boundaries close to a surface for estimating surface movement and/or vibration to obtain information, said surface movement being acoustically and mechanically coupled to a movement of at least one inner element.

4. Device according to any one of claims 1 to 3, wherein said control and/or processing unit is designed for providing a repetition rate of image scanning of at least 5 Hz (Hertz), or of at least 50 Hz, or of at least 500 Hz, or of at least 1000 Hz, or of at least 10000 Hz.

5. Device according to any one of claims 1 to 4, wherein an aperture is provided to obtain an angle-of-arrival resolution of 0.5° or less, preferably of 0.05° or less, perpendicular to an orientation of a line of sight between the at least one antenna or antenna element and the object.

6. Device according to any one of the preceding claims, wherein an aperture is provided by a synthetic aperture obtainable by movement of the at least one antenna or antenna element or by at least one array of multiple antennas or antenna elements.

7. Device according to any one of the preceding claims, wherein
multiple antennas or antenna elements are connected to at least one receiver and/or at least one transmitter

8. Device according to any one of the preceding claims, wherein
- said at least one transmitter and/or said at least one receiver contains at least one MASER (Microwave Amplification by Stimulated Emission of Radiation) and/or
- said at least one comparator comprises at least one electronic mixer and/or at least one electronic filter.

9. Device according to any one of the preceding claims, wherein
the device is designed as a passive radar and the illuminating signal phase is received and phase-referenced by the device, replacing and / or supplementing the phase-coherent TXs in the device.

10. Method for providing data from at least one element of an object using microwaves, preferably microwaves with frequencies between 0.5 GHz to 100 GHz, wherein
- microwaves are emitted from a transmitter in direction to a remote object via an antenna or antenna element,
- microwaves reflected off said object are received by a receiver via an antenna or antenna element,
- emitted microwaves and received microwaves are compared in a comparator,
- at least one property of the emitted microwaves and the received microwaves selected from the group of parameters comprising frequency, phase, polarization and amplitude is analyzed in an analyzer, and
- in a control and/or processing unit two-dimensional images from said compared and analyzed microwaves are repetitively sampled, wherein each pixel of said images contains information about the precise distance change ΔR and/or velocity v of said structural element towards said at least one antenna or antenna element, wherein for each pixel of said images the functions over the time of distance change ΔR(t) and/or velocity v(t) can be calculated, resulting in a repeated spatial reconstruction of said structural element of said object.

11. Method according to claim 10, further **characterized by** being performed using a device having at least one of the features defined in claims 2 to 9.

12. Method according to claim 10 or claim 11, wherein the device according to any one of claims 1 to 9 is used for performing the method.

13. Method according to any one of claims 10 to 12, wherein the reconstruction of a structural element of the living object provided is used for assisting diagnosis of at least one medical condition or disease.

14. Method according to any one of claims 10 to 13, wherein patterns of surface movements are obtained from multiple objects, and where those patterns are used for identification and / or localization of properties of said object and / or diagnosis of at least one medical condition or disease.
